# EUROPEAN PATENT APPLICATION

(11) **EP 3 422 221 A1**
(43) Date of publication of application: **02.01.2019**
(21) Application number: 17178591.8
(22) Date of filing: 29.06.2017
(51) Int. Cl.: G06F 19/00

(54) **ELECTRONIC HEALTH DATA ACCESS CONTROL**

(71) Applicant: Nokia Technologies Oy, 02610 Espoo (FI)
(72) Inventor: ROENNOW, Troels F., Cambridge, Cambridgeshire CB2 9BG (GB); LI, Hongwei, Cambridge, CB4 1RY (GB); BITAULD, David, Cambridge, CB5 8DR (GB); MARTIN LOPEZ, Enrique, Cambridge, CB4 3DN (GB); WABNIG, Joachim, Upper Cambourne, CB23 6AX (GB); PALYUTINA, Karina, Cambridge, CB4 2AW (GB)
(74) Representative: Seppo Laine Oy

(57) **Abstract**

According to an example aspect of the present invention, there is provided a method, comprising: receiving a request for personal health data of a user, obtaining rules for authorizing access to the personal health data on the basis of a smart contract in a distributed network, requesting authorization for accessing the personal health data from one or more authorizers specified by the smart contract, providing received at least one authorization to the distributed network for verifying compliance to the smart contract rules and validating a smart contract transaction authorizing provision of the personal health data.

## Description

### FIELD

The present invention relates to access control to electronic health data and in particular to controlling access to user's health data in case of emergency.

### BACKROUND

Currently, health related data are mainly either maintained by the user (e.g. data produced by wearables), or by different healthcare providers. However, there is a need for a scenario in which all health-related data of a patient/user is aggregated and accessible to him. This may include data coming from different hospitals and health care providers, or even from consumer digital health devices such as smart scales, blood pressure monitors, thermometers, or sleep trackers. This more comprehensive view of a patient's health could then be shared by the user with his doctor or specialist, potentially resulting in better diagnosis and treatment.

Privacy and security of personal health data are paramount in the healthcare industry and services. Currently, sharing of health data between different parts of the healthcare system is usually done by signing a data consent form for each healthcare provider, typically a national or regional health system, or a private service. This fairly static agreement establishes how data is accrued, stored, maintained and shared, and in practice often restricts the data within a particular institution.

Electronic health records (EHRs), also known as electronic medical records (EMRs), collect and aggregate patient and population health information in a digital format. EHRs usually refer to database systems in hospitals and healthcare centres. However, sharing relevant health data is crucial when the patient has EHRs in different institutions or countries. This may become especially important in the case of an emergency, in which the user may be unconscious, and therefore unable to inform about relevant medical information that the emergency services may not be able to access.

### SUMMARY

The invention is defined by the features of the independent claims. Some specific embodiments are defined in the dependent claims.

According to a first aspect of the present invention, there is provided a method, comprising: receiving a request for personal health data of a user, obtaining rules for authorizing access to the personal health data on the basis of a smart contract in a distributed network, requesting authorization for accessing the personal health data from one or more authorizers specified by the smart contract, providing received at least one authorization to the distributed network for verifying compliance to the smart contract rules and validating a smart contract transaction authorizing provision of the personal health data.

According to a second aspect of the present invention, there is provided apparatus comprising at least one processing core, at least one memory including computer program code, the at least one memory and the computer program code being configured to, with the at least one processing core, cause the apparatus at least to: receive a request for personal health data of a user, obtain rules for authorizing access to the personal health data on the basis of a smart contract in a distributed network, request authorization for accessing the personal health data from one or more authorizers specified by the smart contract, provide received at least one authorization to the distributed network for verifying compliance to the smart contract rules and validating a smart contract transaction authorizing provision of the personal health data.

According to a third aspect of the present invention, there is provided a computer program product or a non-transitory computer readable medium having stored thereon a set of computer readable instructions that, when executed by at least one processor, cause an apparatus to at least: receive a request for personal health data of a user, obtain rules for authorizing access to the personal health data on the basis of a smart contract in a distributed network, request authorization for accessing the personal health data from one or more authorizers specified by the smart contract, provide received at least one authorization to the distributed network for verifying compliance to the smart contract rules and validating a smart contract transaction authorizing provision of the personal health data.

According to an embodiment, the smart contract identifies at least one pre-authenticated authorizer, and the received at least one authorization comprises a signed response from the pre-authenticated authorizer.

According to an embodiment, the smart contract identifies at least one emergency authorizer, the authorization is requested from the at least one emergency authorizer in response to the request for personal health data indicating emergency, and the received authorizations comprise a signed response from a trusted entity indicating a response of the emergency authorizer.

According to an embodiment, the request for personal health data indicates non-emergency and is received from a requesting entity not pre-defined as authorized receiver of the personal health data, the authorization is requested from the user in response to the request, the authorization request comprising identification of the requesting entity, and the requesting entity is specified as an authorized receiver after receiving an indication from the user of authorization for the requesting entity.

According to an embodiment, the request for personal health data is provided as a signed request transaction to the distributed network, the authorization is requested from the user in response to validation of the request transaction, and the requesting entity is specified as an authorized receiver in response to verifying a signed transaction from the user indicating authorization for the requesting entity.

According to an embodiment, in response to detecting validation of the smart contract transaction, an authorization to provide the personal health data is provided to a server arranged to access to the personal health data in encrypted form and to a cryptographic key associated with the user for decrypting the encrypted personal health data and encrypt the personal health data for transmission to the requesting entity.

According to an embodiment, a record of the request of the authorization from the at least one authorizer and outcome of the request is stored in the distributed network.

According to an embodiment, the smart contract defines at least one of: which authorizers will be contacted, in which order authorizers are contacted, and authorizer combinations that are required.

According to an embodiment, the distributed network is a blockchain-based private network comprising nodes by healthcare providers and institutions and the steps are carried out by a gateway device arranged to operate as a blockchain node.

### BRIEF DESCRIPTION OF THE DRAWINGS

FIGURE 1 illustrates an example system capable of supporting at least some embodiments of the present invention;
FIGURES 2, 3a, and 3b illustrate methods in accordance with at least some embodiments of the present invention;
FIGURE 4a and 4b illustrate simplified examples of blockchain transaction records in accordance with at least some embodiments of the present invention;
FIGURE 5 illustrates an emergency procedure example in accordance with at least some embodiments of the present invention; and
FIGURE 6 illustrates an apparatus in accordance with at least some embodiments of the present invention.

### EMBODIMENTS

There is now provided a method and apparatus facilitating further automation and secure authorization for providing access to user's health data using a partially decentralized and automatic authorization protocol based on smart contracts stored in a distributed network. The smart contract may specify a set of rules and steps, agreed by the user, and establish who can access the user's heath data in case he is unconscious. A smart contract generally refers to a contract captured in code, which automatically performs the obligations the parties have committed to in the agreement. Smart contracts self-execute the stipulations of an agreement when predetermined conditions are triggered. Smart contract execution usually takes place in a distributed-computing network whose nodes are able to run the code, and therefore, publicly verify its outputs for the corresponding inputs. The smart contract may be user-specific and define the health data access rules and authorizing entities as defined and accepted by the user. The smart contract may comprise executable code that may be stored as part of a transaction in the distributed network.

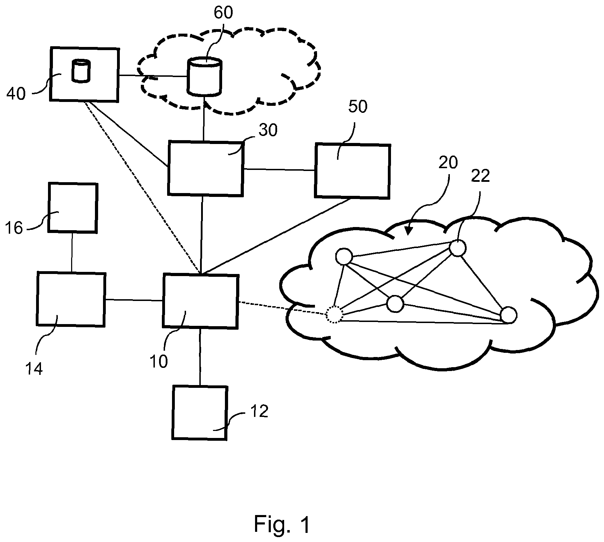

FIGURE 1 illustrates an example system in accordance with at least some embodiments of the present invention. A data storage unit 60, which may be provided on a cloud platform, for example, comprises user's health data encrypted by users' secret key(s). The health data may be stored by the user's device 40 and/or health care provider or institution device. A trusted server 30 may comprise or have access to a copy of the users' secret key(s) and to the health data in the data storage unit 60. A distributed network 20, such as a blockchain platform, comprises identities of users and healthcare providers in the form of an asymmetric key pairs.

A smart contract is stored in the distributed network 20 for authorizing the trusted server 30 to provide (devices of) requesting entities 50 access to a user's health data. A specific application, (central) program or unit/device, such as a gateway 10 as referred hereafter, receives requests for users' health data and communicates with the smart contract on the blockchain. The gateway 10 may host an application sending the requests to appropriate authorizers 12, 14, 16. In the embodiment illustrated in Figure 1 such application or functionality is centralized, but in alternative embodiments it is arranged in a decentralized manner. The gateway 10 may be arranged to manage all or only some health data requests for associated users, such as emergency requests.

The user's device 40 may comprise a secret key used to encrypt the user's health data. The user's device 40 may also comprise a private key of user's asymmetric key pair, allow the user to collect and save different kinds of health data, and/or provide an access to the data for the user when the user is conscious. In case of an emergency and when the user is unconscious, an authorization from the user and his device is not required but a specific emergency protocol as specified by the smart contract is automatically executed.

The trusted server 30 may comprise a copy of the user's secret key. The server 30 may be arranged to, for example by executing a program for this purpose, download encrypted data from the storage unit 60 and decrypt it. Such action or the program can only be triggered by or after permission of the smart contract. In an alternative embodiment, at least some of the gateway/application functionality is hosted in the same device as the trusted server 30.

A pre-authenticated authorizer's device 12 may comprise a private key of the authorizer's asymmetric key pair. The pre-authenticated authorizer's device may comprise an application pushing notifications for authorization requests and signing request response.

A call center 14, or other type of authorization-mediating device or entity, may also have a private-public key pair. The call center may call non-authenticated or emergency authorizers 16 and store a record of their verbal answers. It can be operated by humans or automatized, for example by chat bots. A non-authenticated/emergency authorizer 16 may be known by name, phone number, or has a registered identity as a health services professional, and does not have to be defined in the smart contract.

In an embodiment, a node 10, 22 connected to the distributed network 20 represents an entity that has a stake in a health data management process. The node could correspond to a device of a user/patient, a doctor, a nurse, a care provider, a guardian, a parent, a broker, or other individual. Further, the node could also include other types of entities including a company, an affiliation, a hospital, an organization, a demographic, a community, or other type of entity. The nodes can be different international health care providers and institutions, allowing for a global emergency access system. The smart contract hosted on the distributed network 20 may define the protocol for automatic emergency health data access, and the automatic execution of the smart contract will be verified by the nodes of the corresponding distributed network 20. Such protocol would be compliant with the relevant regulations and described using a programming language for its automatic execution.

The devices 10, 12, 14, 16, 30, 40, 50 may comprise corporate, authority, and/or user devices, such as a server device, a desktop/tablet/laptop computer, smartphone, a machine-to-machine (M2M) device, a set-top box or other suitable electronic device. The system may comprise an administrator or management node, a hub, relay or other kind of intermediate device (not shown) for connecting the devices to further networks or services, such as another distributed or centralized computing system or a cloud service. At least part of the devices are mutually addressable in a suitable way, for example, they may be connected to an internet protocol, IP, network. As one example of a possible variation, in some embodiments the trusted server 30 and/or the requesting entity 50 are connected to the distributed network and may be operate as nodes of the distributed network 22.

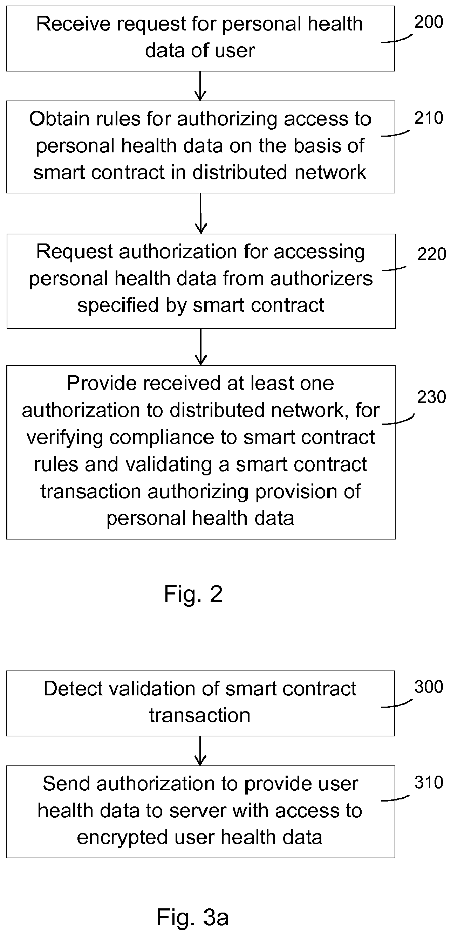

FIGURE 2 illustrates a method according to some embodiments. The method may be implemented in an apparatus for controlling health data access requests, such as the gateway device 10. A request for personal health data of a user is received 200. The request may be a request for authorizing access to the health data and may identify at least the user and the requesting entity 50. The request may also comprise further information, such as which health data (portion) is requested. Rules for authorizing access to the personal health data are obtained 210 on the basis of a smart contract in a distributed network. This may involve accessing the smart contract stored locally in the apparatus or requesting the smart contract information or rules in another apparatus.

Authorization for accessing the personal health data is requested 220 from one or more authorizers specified by the smart contract. At least one authorization is received and provided 230 to the distributed network, for verifying compliance to the smart contract rules and validating a smart contract transaction authorizing provision of the personal health data. The term smart contract transaction is here to be broadly understood to refer to a digitally stored event or information exchange associated with user health data initiated on the basis of the smart contract, and may involve updating the smart contract or an associated record.

In some embodiments, the smart contract identifies at least one pre-authenticated authorizer, and the received at least one authorization comprises a signed response from the pre-authenticated authorizer.

The smart contract may identify at least one emergency authorizer. The authorization may be requested 220 from the emergency authorizer in response to the request for personal health data indicating emergency. Thus, the received authorization(s) may comprise a signed response from a trusted entity indicating a response of the emergency authorizer.

In some embodiments, a record of the request and outcome of the request is stored in the distributed network 20. This may be carried out by the device carrying out the method of Figure 2 after step 230, for example.

Upon verification of the smart contract transaction complying with required criteria for authorizing access to the user's health data as defined by the smart contract, an authorization may be sent to the requesting entity 50 and/or an intermediate device, such as the trusted server 30.

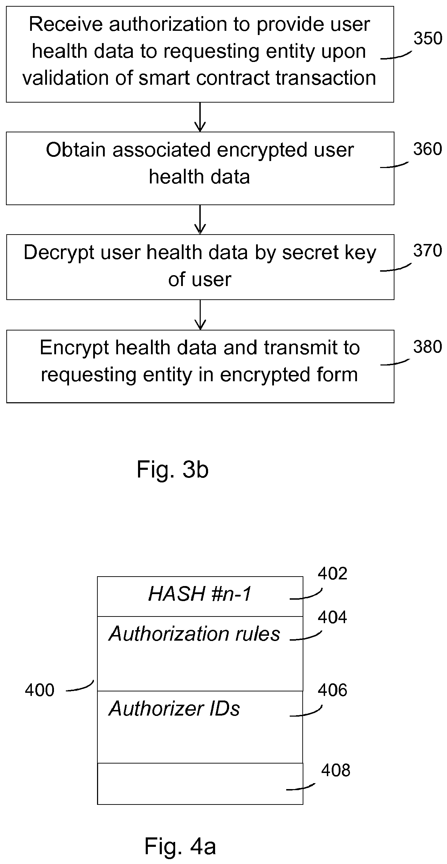

With reference to the methods of FIGUREs 3a and 3b, in response to detecting validation 300 of the smart contract transaction, the gateway 10 may send 310 an authorization to provide the personal health data (for the requesting entity) to the server 30 arranged to access to the health data in encrypted form. In response to such authorization 350, the server obtains 360 the associated encrypted user health data, for example from the storage unit 60 in the cloud.

In some embodiments, public key cryptography is applied in the system. The server 30 has a copy or access to the secret key of the user and decrypts 370 the user data by the user's secret key. The server encrypts 380 the personal health data for transmission to the requesting entity.

If the requesting entity 50 is authorized 350, the trusted server 30 may initialize a protocol to create a secret symmetric key between the server and the requesting entity 50, for example by using Diffie-Hellman key exchange. This secret key can be usable for certain amount of time, and it may even be a one-time password (OTP). In another embodiment, the trusted server 30 may encrypt the data with the public key of the requesting entity 50. The trusted server 30 retrieves 360 and decrypts 370 the relevant piece of user's health data, encrypts it 380, and transmits it to the requesting entity 50.

It is to be noted that the presently disclosed system can be applied to minimize the time to obtain the health record so as to not slow down the emergency procedures. It may be instantly started by one of emergency attendants during their attempt to identify the patient, and it may work behind the scenes to get data ready for the emergency services during emergency procedures. The present system enables an automatic, auditable system for sensitive data retrieval in case of an emergency. Such decentralized system may be arranged to work internationally and it facilitates appropriate patient care by making relevant data automatically available without slowing down medical protocol.

In some embodiments, blockchain-based system and blockchain smart contracts are applied. A blockchain is a distributed computing architecture where every network node executes and records the same transactions, which are grouped into blocks. Only one block can be added at a time, and every block contains a mathematical proof that verifies that it follows in sequence from the previous block. In this way, the blockchain's distributed database is kept in consensus across the whole network, and this is achieved without the need of a central authority. Nodes that maintain and verify the network are incentivized by mathematically enforced economic incentives coded into the protocol. The blockchains can work in different ways, as well as in different scales. Apart from keeping an immutable, comprehensive record of events, to be conveniently acted upon, blockchains offer security in a complex network by accommodating consensus algorithms that are mathematically hard to corrupt.

Thus, the network 20 may be a blockchain network comprising blockchain nodes 22. The gateway 10 may thus operate as a blockchain node, and store local blockchain (BC) database. The devices 10, 70 may host a full blockchain node, making the device a part of the blockchain network.

The nodes 10, 22 are thus configured to manage or at least access health data related transactions in the form of a blockchain. Each block in the chain includes one or more transactions that further incorporate information representing health data access control related transaction and/or smart contract information by at least some of the nodes. Each block within the blockchain may be identified by a hash, generated e.g. using an SHA256 cryptographic hash algorithm, or some other cryptographic hash algorithm, on the header of the block. Each block also references a previous block, known as the parent block, through a previous block hash field in the block header. In other words, each block contains the hash of its parent inside its own header. The sequence of hashes linking each block to its parent creates a chain going back all the way to the first block ever created, known as the genesis block.

At least some of the nodes 10, 22 may generate blockchain transaction data relating to the node and hash the data using a cryptographic hashing function, to create a cryptographic hash block. The transaction data, such as a smart contract or health data access request/authorization data item, may be stored at a node that adds a hash of an asymmetric encryption on to generate a hash block. Nodes may validate and commit transactions in order to reach consensus. Each node may have their own copy of the ledger which is in some embodiments permission-controlled, so participants see only appropriate transactions.

A node establishing the next block may be known as a miner node. A miner node may compile a set of transactions, which it receives from the broadcasts, for the next block, and search for a proof-of-work code that covers all the transactions in the set of transactions for the next block. For example, the proof-of-work code may be a numerical value, with which the contents of the next block, that is, the set of transactions, hashes to a value that is less than a threshold. More generally, there may be a target area of an output space of a hash function, wherein the target space need not be in the low end of the target space. The smaller the target area is, the more difficult it is to discover the proof-of-work. Once a miner discovers the proof-of-work, it can publish the block, which other nodes of the system will then add to the block chain as the new most recent established block.

In case the miner node discovers a proof-or-work based on an incomplete set of transactions, for example if some transactions did not reach the miner node, other nodes in the network will not accept the block into the blockchain, and it will be excluded from a consensus version of the blockchain in the system.

In some embodiments, the request 200 for personal health data is a signed blockchain transaction to the blockchain network 20. The authorization may be requested 220 from the user or the authorizers in response to validation of the request transaction.

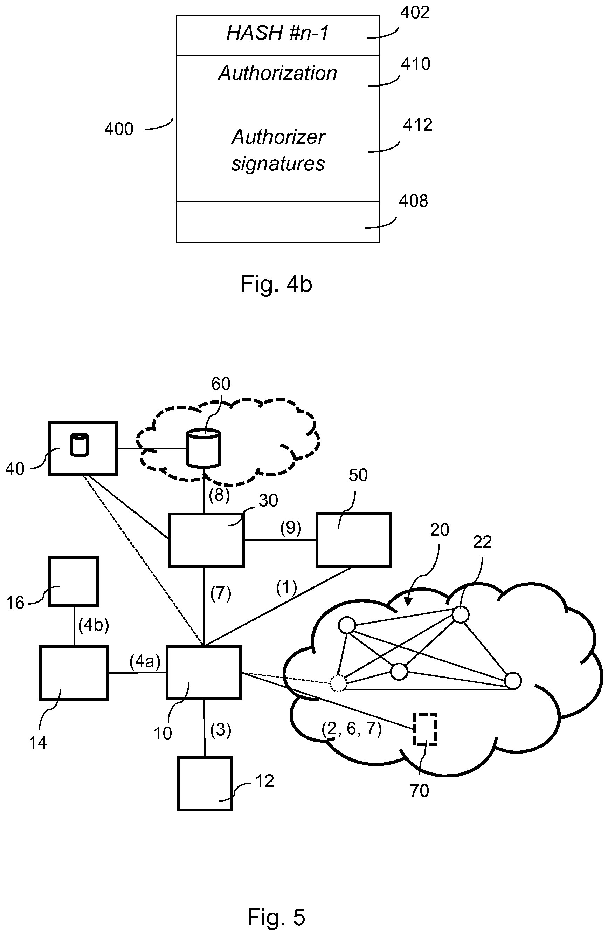

FIGUREs 4a and 4b illustrate simplified examples of a blockchain transaction records n 400 that may be generated by a node 10, 22. The transaction record comprises a hash pointer 402 to the previous block *n*-*1* of the chain. The transaction record of Figure 4a may be a smart contract transaction record and may comprise smart contract data, such as authorization rules 404 and authorizer IDs 406.

The IDs are generally used to represent actors in the system, such as users/patients, doctors, gateways, authorizers, requesting entities etc. In the preferred implementation the IDs are public keys, each of which has a corresponding private key. The person in possession of the private key is the sole owner of the ID, thus making them the only person capable of signing transactions involving that ID. The signature may be generated with a smartcard, a wearable, a token, a private key on a computer, mobile terminal or any other device capable of making a digital signature.

FIGURE 4b illustrates a transaction record prepared as outcome of a user health data access request. The record may comprise data indicating authorization 410 for the requesting entity to access the health data (with appropriate IDs) and authorizer signatures. The records may also comprise also further information element(s) 408, such as a timestamp.

### Non-emergency usage of the system (conscious user)

In some embodiments, the request for personal health data indicates non-emergency and is received 200 from a requesting entity 50 not pre-defined as authorized receiver of the personal health data. The authorization is requested from the user device 40, wherein the authorization request comprises an identification of the requesting entity. The requesting entity is specified as an authorized receiver upon receiving a response from the user device 40 indicating authorization for the requesting entity. A further example of such non-emergency operations is provided below.

In this example scenario, the smart contract involves direct authorization from the user for each new requesting entity 50, such as a new doctor or new health institution. The new requesting entity may directly or indirectly emit a new signed transaction to the smart contract on the blockchain, requesting to be added to the list of authorized identities for the user's health data. The smart contract specifies that in response response to validation of the request transaction, the user must emit a signed transaction accepting or rejecting the request. This may be considered as the equivalent of signing an initial data consent form. Thus, the request may be sent to the user device 40.

In response to obtaining (a validated) transaction indicating the user's acceptance, a blockchain identity of the new requesting entity may be added to a list in the internal storage of the smart contract that defines the automatic access control of the user's health data. Any identity on this list can request user's data to the trusted server 30. In an embodiment this is triggered by a data-access transaction to the smart contract, leaving a record on the blockchain which is caught by the trusted server 30 (constantly listening to BC transactions). In another embodiment a signed request is sent directly to the trusted server 30 (not leaving a record on the BC), which verifies the signature using the list of authorized parties in the internal storage of the smart contract. This verification may leave a record on the BC. The server 30 may then proceed to provide the health data for the requesting entity as illustrated above in connection with Figure 3b.

### Emergency usage of the system

A further example of an emergency protocol is provided below, where the user may be unconscious. In this example scenario, a list or other type of record of authorizers 12, 16 exists in the internal storage of the smart contract. The authorizers and/or intermediating devices 10, 14 on behalf of the authorizers may be arranged to emit signed authorization transactions to the blockchain to allow access to user's 40 health data for new requesting entities 50 on behalf of the user. Such record may be created at the time of contract deployment and specifies people, contact details, and categories than can then be used by the automatic rules programmed in the smart contract.

In an emergency, an entity 50 needing the data, such as an accident and emergency (A&E) doctor, identifies the patient (documentation, biometry), and sends a data access request to the trusted server 30 or the gateway 10. If the requesting entity 50 has been earlier authorized by the user, for example they may belong to a national health service that the patient has used earlier, they will be able to access the data as described in the previous example scenario. Otherwise an emergency request transaction is sent to the smart contract.

In the case the emergency request transaction is required, the set of rules described in the smart contract defines which authorizers will be contacted, in which order authorizers are contacted, and/or authorizer combinations that are required for authorization. For example, the smart contract may define for the user the following rules: contact next of kin first -> if not accessible, contact specified general practitioner (GP) -> if no answer, get approval from at least 2 doctors.

If the rules are followed, the smart contract allows and thus triggers the delivery of the health data to the requester. One of the challenges is to construct a proof that the rules are indeed followed.

In a first example case, the authorizer 12 is pre-authenticated by being a part of an authenticated or validated authorizers list in the internal storage of the smart contract. This means that the identity of the authorizer has been established, e.g. using a private-public key pair, the public key being then added to said list. In this case the authorizer can sign a request sent by the system. In some embodiments, the authorizer has an app related to the system that notifies them that their authorization is needed and performs the signature. If a sufficient number of authorizers (as predefined by the smart contract) give their approval, the smart contract may initiate approval for the trusted server 30, which triggers release of the data upon receiving 350 the authorization.

In a second example case, the first tier of authorizers may not be able to respond, and emergency authorizers, or non-authenticated authorizers 16 (not listed in the internal storage of pre-authenticated authorizers) may be required. Following the embodiment of the first case, this could mean that they do not have a registered public key or the app. Emergency authorizers would typically be doctors of a national health institution, which could be reached by calling the nearest emergency services. The call center 14 would be in charge of making this call and their verbal answer may be recorded to produce a proof of this call such as a hash of the sound file to be stored on the blockchain. The call center, like the pre-authenticated authorizer, may have the capability to sign the request with their BC identity (asymmetric key pair) on behalf of the emergency authorizer. For the sake of rapidity in the emergency response, the call center is temporarily trusted to interpret the verbal answer of the authorizer. However, in the unordinary case of a dispute after the emergency procedures, the sound records serve as proof and can be manually checked.

If when following the protocol, a given authorizer 16 is not accessible, a record may be created on the blockchain showing that an attempt has been made to contact him/her. This can be done by requiring a response within a time limit. If the request to the next of kin receives no response within this limit, the central application automatically sends a signed notification that the request has not been responded by the authorizer, so that the trusted server knows the rules are followed and the next authorizer, such as a GP, can be contacted.

Latency is crucial, so all the requests can be sent 220 out at once by the gateway 10, outside the blockchain and the trusted server. The gateway may be arranged to collect proof of its actions, such as collecting digital signatures and hashes of verbal answers. Once a sufficient approval number is collected, the verification 230 of the compliance to the smart contract may be carried out in one go. In other words the participants of the blockchain platform will have to validate a final smart contract transaction. The result of this smart contract transaction is the request/authorization 350 to the trusted server 30 to communicate the appropriate data to the requester.

With reference to FIGURE 5, an example emergency procedure on the basis of blockchain smart contract, also indicated by block 70, is now illustrated: An emergency care provider, such as an ambulance paramedic or an A&E doctor, needs data relevant to the rescue of an unconscious user.
(1) The care provider as requesting entity 50 sends a request to the system to have access to data.
(2) The gateway 10 checks 210 what are the rules of the emergency smart contract.
(3) The gateway 10 requests 220 authorization from the pre-authenticated authorizers 12, for example through a specific app in authorizers' devices.
(4) For non-authenticated authorizers 16, the gateway uses (4a) a call center 14 to request authorization. The call center contacts (4b) the non-authenticated authorizer(s) 16 records the answer(s).
(5) The call center signs the request on the behalf of the authorizers if they give their consent and sends them to the gateway 10.
(6) The required signatures and hashes are communicated 230 to the blockchain network 20. The nodes of the blockchain network verify the transactions, and hence, that authorizations have been given according to the rules. For such verification to be fast, a private network running practical byzantine fault tolerance (PBFT) consensus, in which the nodes are healthcare institutions.
(7) If the rules have been followed, the smart contract 70 gives authorization for the trusted server 30 to release the relevant data. The authorization may be provided via the gateway 10 in case the server 30 is not connected to the blockchain network.
(8) The trusted server 30 downloads the relevant data and decrypts the data with the copy of the secret key.
(9) The trusted server 30 sends the data to the requester in an encrypted form. This may be arranged similarly as illustrated above for the non-emergency usage of the system.

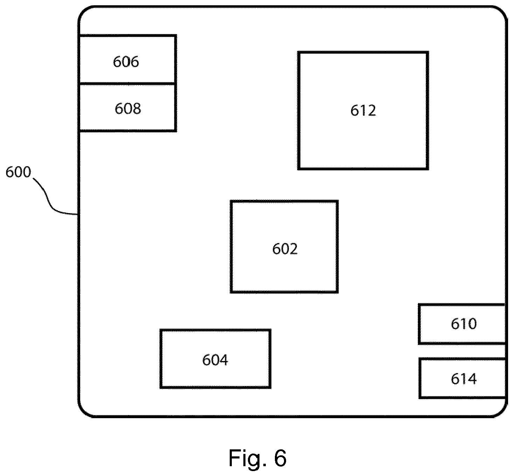

FIGURE 6 illustrates an example apparatus capable of supporting at least some embodiments of the present invention. Illustrated is a device 600, which may comprise a communications device arranged to operate as a node in a distributed network. The device may be arranged to carry out at least some of the embodiments related to managing access to personal health data on the basis of a smart contract as illustrated above. The device may include one or more controllers configured to carry out operations in accordance with at least some of the embodiments illustrated above, such as some or more of the features illustrated in connection with Figures 1 to 5. The device may operate as the gateway 10 and/or the trusted server 30.

Comprised in the device 600 is a processor 602, which may comprise, for example, a single- or multi-core processor wherein a single-core processor comprises one processing core and a multi-core processor comprises more than one processing core. The processor 602 may comprise more than one processor. The processor may comprise at least one application-specific integrated circuit, ASIC. The processor may comprise at least one field-programmable gate array, FPGA. The processor may be means for performing method steps in the device. The processor may be configured, at least in part by computer instructions, to perform actions.

The device 600 may comprise memory 604. The memory may comprise random-access memory and/or permanent memory. The memory may comprise at least one RAM chip. The memory may comprise solid-state, magnetic, optical and/or holographic memory, for example. The memory may be at least in part accessible to the processor 602. The memory may be at least in part comprised in the processor 602. The memory 604 may be means for storing information. The memory may comprise computer instructions that the processor is configured to execute. When computer instructions configured to cause the processor to perform certain actions are stored in the memory, and the device in overall is configured to run under the direction of the processor using computer instructions from the memory, the processor and/or its at least one processing core may be considered to be configured to perform said certain actions. The memory may be at least in part comprised in the processor. The memory may be at least in part external to the device 600 but accessible to the device. For example, control parameters affecting operations related to managing access to health data and actions associated with the smart contract and associated transactions may be stored in one or more portions of the memory and used to control operation of the apparatus. Further, the memory may comprise device-specific cryptographic information, such as secret and public key of the device 600.

The device 600 may comprise a transmitter 606. The device may comprise a receiver 608. The transmitter and the receiver may be configured to transmit and receive, respectively, information in accordance with at least one wired or wireless, cellular or non-cellular standard. The transmitter may comprise more than one transmitter. The receiver may comprise more than one receiver. The transmitter and/or receiver may be configured to operate in accordance with global system for mobile communication, GSM, wideband code division multiple access, WCDMA, long term evolution, LTE, IS-95, wireless local area network, WLAN, Ethernet and/or worldwide interoperability for microwave access, WiMAX, standards, for example. The device 600 may comprise a near-field communication, NFC, transceiver 610. The NFC transceiver may support at least one NFC technology, such as NFC, Bluetooth, Wibree or similar technologies.

The device 600 may comprise user interface, UI, 612. The UI may comprise at least one of a display, a keyboard, a touchscreen, a vibrator arranged to signal to a user by causing the device to vibrate, a speaker and a microphone. A user may be able to operate the device via the UI, for example to accept incoming and outgoing communications, adjust the operation of the device, to manage other devices and remotely stored information via the device 600, browse the Internet, to manage digital files stored in the memory 604 or on a cloud accessible via the transmitter 606 and the receiver 608, or via the NFC transceiver 610.

The device 600 may comprise or be arranged to accept a user identity module or other type of memory module 614. The user identity module may comprise, for example, a subscriber identity module, SIM, and/or a personal identification IC card installable in the device 600. The user identity module 614 may comprise information identifying a user of the device 600, or the user's subscription or role in the system. The user identity module 614 may comprise cryptographic information usable to verify the identity of a user of device 600 and/or to facilitate encryption and decryption of data and communication effected via the device 600, such as the private and/or public keys as illustrated above.

The processor 602 may be furnished with a transmitter arranged to output information from the processor, via electrical leads internal to the device 600, to other devices comprised in the device. Such a transmitter may comprise a serial bus transmitter arranged to, for example, output information via at least one electrical lead to memory 604 for storage therein. Alternatively to a serial bus, the transmitter may comprise a parallel bus transmitter. Likewise the processor may comprise a receiver arranged to receive information in the processor, via electrical leads internal to the device 600, from other devices comprised in the device 600. Such a receiver may comprise a serial bus receiver arranged to, for example, receive information via at least one electrical lead from the receiver 608 for processing in the processor. Alternatively to a serial bus, the receiver may comprise a parallel bus receiver.

The device 600 may comprise further devices not illustrated in Figure 6. For example, the device may comprise at least one digital camera. Some devices may comprise a back-facing camera and a front-facing camera. The device may comprise a fingerprint sensor arranged to authenticate, at least in part, a user of the device. In some embodiments, the device lacks at least one device described above. For example, some devices may lack the NFC transceiver 610 and/or the user identity module 614.

The processor 602, the memory 604, the transmitter 606, the receiver 608, the NFC transceiver 610, the UI 612 and/or the user identity module 614 may be interconnected by electrical leads internal to the device 600 in a multitude of different ways. For example, each of the aforementioned devices may be separately connected to a master bus internal to the device, to allow for the devices to exchange information. However, as the skilled person will appreciate, this is only one example and depending on the embodiment various ways of interconnecting at least two of the aforementioned devices may be selected without departing from the scope of the present invention.

It is to be understood that the embodiments of the invention disclosed are not limited to the particular structures, process steps, or materials disclosed herein, but are extended to equivalents thereof as would be recognized by those ordinarily skilled in the relevant arts. It should also be understood that terminology employed herein is used for the purpose of describing particular embodiments only and is not intended to be limiting.

References throughout this specification to one embodiment or an embodiment means that a particular feature, structure, or characteristic described in connection with the embodiment is included in at least one embodiment of the present invention. Thus, appearances of the phrases "in one embodiment" or "in an embodiment" in various places throughout this specification are not necessarily all referring to the same embodiment. The skilled person will appreciate that above-illustrated embodiments may be combined in various ways. Embodiments illustrated in connection with Figures 1 to 5 may be taken in isolation or further combined together.

Various embodiments and examples of the present invention may be referred to herein along with alternatives for the various components thereof. It is understood that such embodiments, examples, and alternatives are not to be construed as de facto equivalents of one another, but are to be considered as separate and autonomous representations of the present invention.

Furthermore, the described features, structures, or characteristics may be combined in any suitable manner in one or more embodiments. In the preceding description, numerous specific details are provided, such as examples of lengths, widths, shapes, etc., to provide a thorough understanding of embodiments of the invention. One skilled in the relevant art will recognize, however, that the invention can be practiced without one or more of the specific details, or with other methods, components, materials, etc. In other instances, well-known structures, materials, or operations are not shown or described in detail to avoid obscuring aspects of the invention.

While the forgoing examples are illustrative of the principles of the present invention in one or more particular applications, it will be apparent to those of ordinary skill in the art that numerous modifications in form, usage and details of implementation can be made without the exercise of inventive faculty, and without departing from the principles and concepts of the invention. Accordingly, it is not intended that the invention be limited, except as by the claims set forth below.

The verbs "to comprise" and "to include" are used in this document as open limitations that neither exclude nor require the existence of also un-recited features. The features recited in depending claims are mutually freely combinable unless otherwise explicitly stated. Furthermore, it is to be understood that the use of "a" or "an", that is, a singular form, throughout this document does not exclude a plurality.

### INDUSTRIAL APPLICABILITY

At least some embodiments of the present invention find industrial application in communications.

### ACRONYMS LIST

- ASIC: Application-specific integrated circuit
- BC: Blockchain
- EHR: Electronic health record
- EMR: Electronic medical record
- FPGA: Field-programmable gate array
- HTTPS: Hypertext transfer protocol secure
- GSM: Global system for mobile communication
- LTE: Long term evolution
- NFC: Near-field communication
- PBFT: Practical byzantine fault tolerance
- SIM: Subscriber identity module
- UI: User interface
- WCDMA: Wideband code division multiple access,
- WiMAX: Worldwide interoperability for microwave access
- WLAN: Wireless local area network

## Claims

1. A method, comprising:
- receiving a request for personal health data of a user,
- obtaining rules for authorizing access to the personal health data on the basis of a smart contract in a distributed network,
- requesting authorization for accessing the personal health data from one or more authorizers specified by the smart contract,
- providing received at least one authorization to the distributed network for verifying compliance to the smart contract rules and validating a smart contract transaction authorizing provision of the personal health data.

2. The method of claim 1, wherein the smart contract identifies at least one pre-authenticated authorizer, and the received at least one authorization comprises a signed response from the pre-authenticated authorizer.

3. The method of claim 1 or 2, wherein the smart contract identifies at least one emergency authorizer, the authorization is requested from the at least one emergency authorizer in response to the request for personal health data indicating emergency, and the received authorizations comprise a signed response from a trusted entity indicating a response of the emergency authorizer.

4. The method of claim any preceding claim, wherein the request for personal health data indicates non-emergency and is received from a requesting entity not pre-defined as authorized receiver of the personal health data, the authorization is requested from the user in response to the request, the authorization request comprising identification of the requesting entity, and the requesting entity is specified as an authorized receiver after receiving an indication from the user of authorization for the requesting entity.

5. The method of any claim 4, wherein the request for personal health data is provided as a signed request transaction to the distributed network,
the authorization is requested from the user in response to validation of the request transaction, and
the requesting entity is specified as an authorized receiver in response to verifying a signed transaction from the user indicating authorization for the requesting entity.

6. The method of claim any preceding claim, wherein, in response to detecting validation of the smart contract transaction, an authorization to provide the personal health data is provided to a server arranged to access to the personal health data in encrypted form and to a cryptographic key associated with the user for decrypting the encrypted personal health data and encrypt the personal health data for transmission to the requesting entity.

7. The method of claim any preceding claim, wherein a record of the request of the authorization from the at least one authorizer and outcome of the request is stored in the distributed network.

8. The method of claim any preceding claim, wherein the smart contract defines at least one of: which authorizers will be contacted, in which order authorizers are contacted, and authorizer combinations that are required.

9. The method of claim any preceding claim, wherein the distributed network is a blockchain-based private network comprising nodes by healthcare providers and institutions and the steps are carried out by a gateway device arranged to operate as a blockchain node.

10. An apparatus comprising:
- means for receiving a request for personal health data of a user,
- means for obtaining rules for authorizing access to the personal health data on the basis of a smart contract in a distributed network,
- means for requesting authorization for accessing the personal health data from one or more authorizers specified by the smart contract,
- means for providing received at least one authorization to the distributed network for verifying compliance to the smart contract rules and validating a smart contract transaction authorizing provision of the personal health data.

11. The apparatus of claim 10, wherein the apparatus comprises means for carrying out the method according to any one of claims 2 to 9.

12. A health data management system, comprising the apparatus according to claim 10 or 11.

13. A computer program comprising a set of computer readable instructions that, when executed by at least one processor, cause an apparatus to perform the method of any one of claims 1 to 9.
